## Europäisches Patentamt
## European Patent Office
## Office européen des brevets

(19)

(11) Veröffentlichungsnummer: **0 106 968**
**A1**

(12) **EUROPÄISCHE PATENTANMELDUNG**

(21) Anmeldenummer: 83108226.8

(22) Anmeldetag: 20.08.83

(51) Int. Cl.³: **C 07 C 61/24**, C 07 C 51/567,
C 07 C 51/353, C 07 C 69/75,
C 07 C 67/333

(30) Priorität: 22.10.82 DE 3239055

(71) Anmelder: **CHEMISCHE WERKE HÜLS AG, - RSP
Patente / PB 15 - Postfach 13 20, D-4370 Marl 1 (DE)**

(43) Veröffentlichungstag der Anmeldung: **02.05.84
Patentblatt 84/18**

(72) Erfinder: **Gude, Fritz, Dr., Wilhelmstrasse 4,
D-4690 Herne 2 (DE)**
Erfinder: **Haferkorn, Herbert, Horster Strasse 528,
D-4250 Bottrop (DE)**

(84) Benannte Vertragsstaaten: **CH DE FR GB LI NL**

(54) **Verfahren zur Herstellung von Isomeren der 4-Methyltetrahydrophthalsäurederivate und/oder
Tetrahydrophthalsäurederivate, insbesondere deren Anhydride.**

(57) Ein Verfahren zur Herstellung von Isomeren der 4-Methyltetrahydrophthalsäurederivate und/oder Tetrahydrophthalsäurederivate, insbesondere deren Anhydride, gegebenenfalls im Gemisch mit Methyl-hexahydrophthalsäure-Verbindungen und/oder Hexahydrophthalsäure-Verbindungen, wobei man die 4-Methyl-4-tetrahydrophthalsäurederivate und/oder 4-Tetrahydrophthalsäurederivate bei Temperaturen von 130 bis 250 °C, insbesondere bei 150 bis 220 °C, mit aktivierten Bleicherden behandelt.

EP 0 106 968 A1

0106968

CHEMISCHE WERKE HÜLS AG

- RSP PATENTE -

O.Z. 3847-H

Verfahren zur Herstellung von Isomeren der 4-Methyl-
tetrahydrophthalsäurederivate und/oder Tetrahydrophthalsäurederivate, insbsondere deren Anhydride

Bei gewöhnlicher Temperatur flüssige Dicarbonsäureanhydride
bzw. Dicarbonsäureanhydridgemische sind z.B. wegen der
leichten und bequemen Verarbeitbarkeit begehrte Härter für
Epoxidharze. Je nach ihrer Konstitution üben sie einen starken Einfluß auf die Eigenschaften der durch sie gehärteten
Harze aus. So haben sich hinsichtlich z.B. der Wärmestandsfestigkeiten und der Chemikalienbeständigkeiten dieser
Massen hydroaromatische Carbonsäureanhydride bzw. ihre Gemische ohne oder mit kurzen Alkylgruppen als Härter bewährt.
Beispiele sind Tetrahydrophthalsäureanhydride, Hexahydrophthalsäureanhydride, Methyl-tetrahydrophthalsäureanhydride,
Methyl-hexahydrophthalsäureanhydride, Dimethyl-tetrahydrophthalsäureanhydride und Dimethyl-hexahydrophthalsäureanhydride.

Die vollständig kernhydrierten Anhydride, wie z.B. Hexahydrophthalsäureanhydride und Methyl-hexahydrophthalsäureanhydride erweisen sich chemisch zwar indifferenter als die ungesättigten Anhydride, neigen aber viel stärker zur Hydrolyse
bei Anwesenheit von Feuchtigkeit und als Folge zur Abscheidung von in den Anhydriden schwer löslichen Dicarbonsäuren.

Durch Bildung der Carboxylgruppen aus den Anhydriden der
Härter tritt eine unkontrollierbare Reduktion des "potlife"
im Gemisch mit Epoxidharzen ein.

In der DE-AS 2 159 420 wird dargelegt, wie man durch Anlagerung von trans-Piperylen an Maleinsäureanhydrid und Erhitzen des Reaktionsproduktes auf 150-250°C ein flüssiges Isomerengemisch des 3-Methyl-tetrahydrophthalsäureanhydrids erhalten kann. Nun steht leider reines trans-Piperylen in technischen Mengen nicht zur Verfügung und müßte erst durch aufwendige und umständliche Reinigungsoperationen, z.B. aus einem $C_5$-Crackschnitt, gewonnen werden.

Dagegen sind bekanntlich reines Isopren und Butadien Produkte der Großchemie. Die Reaktionen von Isopren mit Maleinsäureanhydrid zu 4-Methyl-$\Delta$4-tetrahydrophthalsäureanhydrid und Butadien zu $\Delta$4-Tetrahydrophthalsäureanhydrid, z.B. durch Einleiten der gasförmigen Diene in die Maleinsäureanhydridschmelze, ist lange bekannt. Die Isomerisierungen der bei 68°C bzw. 102°C schmelzenden Addukte zu flüssigen Anhydridgemischen lassen sich rein thermisch nicht ausreichend durchführen. Deshalb werden in der US-PS 29 59 599 für 4-Methyl-$\Delta$4-tetrahydrophthalsäureanhydrid anorganische Verbindungen, wie Schwefelsäure, Phosphorsäure, ihre Anhydride, Chlorsulfosäure, Phosphorchloride oder Natriumbisulfat als Katalysatoren vorgeschlagen, die aber bekanntlich nur in speziellen säurebeständigen Reaktoren zum Einsatz kommen können. Außerdem muß das Reaktionsprodukt noch zusätzlich entsäuert werden, wozu Neutralisation und/oder Destillation notwendig ist. Die gleichen Nachteile besitzt das mit Phosphorpentoxid isomerisierte $\Delta$-4-Tetrahydrophthalsäureanhydrid nach GB-PS 914 463.

Die DE-AS 15 95 458 empfiehlt, die Isomerisierung von Methyltetrahydrophthalsäureanhydriden mit fein verteilten Palladium- oder Ruthenium-Katalysatoren zu flüssigen Anhydridgemischen mit guten Härtereigenschaften durchzuführen. Die Isomerisierung von z.B. $\Delta$4-Tetrahydrophthalsäureanhydriden mit gleichen Katalysatoren wird durch US-PS 2 764 597 geschützt. Diese Verfahren haben den Nachteil, daß die empfindlichen Edelmetalle die Isomerisierung - je nach Gehalt an Katalysatorgiften im technischen Reaktionsprodukt - verschieden stark und unkontrollierbar tätigen. Aus diesem Grun-

0106968

de kann die Reaktion bei mehrfachem Einsatz dieser kostspieligen Katalysatoren erst recht nicht mehr übersehen werden. So kristallisierte unvorhergesehen beim Einsatz von Methyl-Δ4-tetrahydrophthalsäureanhydrid in einigen Fällen nach einer gewissen Standzeit das mit dem Isomerengemisch unverträgliche Methyl-phthalsäureanhydrid aus, das sich als Nebenreaktion unter der Wirkung der Edelmetallkatalysatoren durch Disproportionierung des Methyltetrahydrophthalsäureanhydrids neben Methyl-hexahydrophthalsäureanhydrid gebildet hatte.

Es wurde nun überraschend gefunden, daß handelsübliche, zur Entfernung der Kationen mit Säuren nach bekannten Verfahren gewaschene Bleicherden vom Typ Montmorillonit 4-Methyl-Δ4-tetrahydrophthalsäureanhydrid und/oder Δ4-Tetrahydrophthalsäureanhydrid in flüssige lagerstabile Gemische zu isomerisieren vermögen, wobei eine Disproportionierung unter Bildung von aromatischen Säureanhydriden nicht eintritt.

Im Gegensatz zum Palladium- bzw. Rutheniumkatalysator ist der Isomerisierungsgrad bei Verwendung der erfindungsgemäß eingesetzten Katalysatoren von Katalysatorgiften unabhängig und gut reproduzierbar. Die notwendige Reaktionszeit, um bei vergleichbaren Temperaturen zu flüssigen Gemischen zu gelangen, erweist sich besonders beim 4-Methyl-Δ4-tetrahydrophthalsäureanhydrid überraschenderweise erheblich kürzer als bei Verwendung der Katalysatoren nach US-PS 2 959 599.

Wie bei den Anhydriden beschrieben, ist die Verschiebung der Doppelbindung mit den Katalysatoren gemäß dieser Erfindung auch mit anderen Derivaten der 4-Methyl-tetrahydrophthalsäure und/oder Tetrahydrophthalsäure möglich, z.B. den Estern.

Gegenstand der Erfindung ist daher ein Verfahren zur Herstellung von Isomeren der 4-Methyl-tetrahydrophthalsäurederivate und/oder Tetrahydrophthalsäurederivate, insbesondere deren Anhydride, gegebenenfalls im Gemisch mit Methyl-hexahydrophthalsäure-Verbindungen und/oder Hexahydrophthalsäure-Verbindungen, das dadurch gekennzeichnet ist, daß man die 4-Methyl-$\Delta$4-tetrahydrophthalsäurederivate und/oder $\Delta$4-Tetrahydrophthalsäurederivate mit aktivierten Bleicherden behandelt.

Als Bleicherden eignen sich die praktisch neutralen bis sauren Typen, z.B. Bentonite, Fullererde, Floridaerde, Walkererde, deren Herstellung und Zusammensetzung z.B. im Römpp "Chemielexikon", 5.Auflage (1962), S. 554-555, Houben-Weyl "Methoden der org.Chemie", Bd. 4, Teil 2 (1955), S. 149 und im "Ullmanns Enzyklopädie der technischen Chemie", 3.Auflage, Bd. 4 (1953), S. 544 beschrieben sind. Dabei hat sich eine Menge von 3-15 Gew.%, bezogen auf die Reaktionsmischung, als optimal erwiesen.

Die Isomerisation kann bei Temperaturen von 130-250°C durchgeführt werden.

Die Reaktion des bei 68°C schmelzenden 4-Methyl-$\Delta$4-tetrahydrophthalsäureanhydrids und/oder des bei 102°C schmelzenden $\Delta$4-Tetrahydrophthalsäureanhydrids zu lagerbeständigen flüssigen Isomerengemischen erfolgt mit dem erfindungsgemäß eingesetzten Katalysator schonend, überraschend rasch und unter vergleichbaren Bedingungen, besonders beim 4-Methyl-$\Delta$4-tetrahydrophthalsäureanhydrid,erheblich schneller als mit den Katalysatoren gemäß US-PS 2 959 599 bzw. GB-PS 914 463. Die Bildung von Polymeren, vor allem bedingt durch die Empfindlichkeit der Substanz gegenüber starken Säuren, wird durch Verwendung des erfindungsgemäß eingesetzten Katalysators praktisch ganz zurückgedrängt.

Es war nicht vorauszusehen, daß die Isomerisationsreaktion von 4-Methyl-Δ4-tetrahydrophthalsäureanhydrid und/oder Δ4-Tetrahydrophthalsäureanhydrid mit den erfindungsgemäß eingesetzten Katalysatoren in so kurzer Zeit und ohne Bildung von Nebenprodukten durchgeführt werden kann. So ist es nun möglich geworden, das Reaktionsprodukt nach Abtrennen der Bleicherdekatalysatoren auf mechanischem Weg auch ohne Destillation als Härter für Epoxidharze zu verwenden. Da die Katalysatoren Feuchtigkeit abspalten können, sollte man sie vor dem Einsatz etwa in der Höhe der Isomerisationstemperatur trocknen. Eine merkliche Einbuße an Isomerisationsaktivität konnte dadurch nicht festgestellt werden.

Es ist möglich, die Bleicherdekatalysatoren für die Isomerisation mehrfach zu verwenden. Wenn sie ausgebraucht sind, sind die Erden, evtl. nach Abrösten, für die Umwelt unschädlich.

Selbstverständlich ist es auch möglich, das isomerisierte 4-Methyl-tetrahydrophthalsäureanhydrid und/oder Tetrahydrophthalsäureanhydrid zusammen mit anderen geeigneten Anhydridhärtern, wie z.B. Hexahydrophthalsäureanhydrid, Methyl-hexahydrophthalsäureanhydriden und 3-Methyl-Δ4-tetrahydrophthalsäureanhydrid bzw. sein Isomerengemisch als Härter für Epoxidharze einzusetzen.

Natürlich kann das flüssige, isomerisierte 4-Methyl-tetrahydrophthalsäureanhydrid und/oder Tetrahydrophthalsäureanhydrid auch im Vakuum destilliert werden, wobei die Bildung eines festen gummiartigen polymeren Rückstandes zunächst manchmal nicht ganz vermieden werden kann. Es ist aber möglich, die Bildung der Polymeren praktisch ganz zu unterdrücken, wenn man Isomerisierung und Destillation in stabilen, geeigneten Anhydriden, wie z.B. Hexahydrophthalsäureanhydrid und/oder Methyl-hexahydrophthalsäureanhydriden, als reaktive Verdünnungsmittel durchführt. Eine besonders elegante Ausführungsweise zur Herstellung solcher Lösungen des isomerisierten 4-Methyl-tetrahydrophthalsäureanhydrids und/oder Tetrahydrophthalsäureanhydrids ist eine gemeinsame Vakuumdestillation, wobei letzte Reste von Feuchtigkeit über Kopf abgenommen werden können. Das Destillat besteht dann aus einer lagerstabilen flüssigen reaktiven Lösung. Eine eventuelle Vorbehandlung des Katalysators durch spezielles Trocknen erübrigt sich in diesem Fall.

Beispiele
---------

Die in den folgenden Beispielen aufgeführten Wassergehalte der Anhydride wurden nach Karl-Fischer gemessen. Die Säurebestimmung der Substanz erfolgte als Lösung in Acetonitril nach Zugabe von Bariumperchlorat durch potentiometrische Titration mit Tri-n-propylamin(Elektroden: Platin/Kalomel) nach E.J.Greenhow, R.L.Parry Jones, Analyst 97 (1972), 346-351 und H.W. Wharton, Anal.Chem. 37 (1965), 730-733.

A) Isomerisation von 4-Methyl-Δ4-tetrahydrophthalsäureanhydrid (4-M-Δ4-THPSA)

Das durch Anlagerung von Isopren an Maleinsäureanhydrid nach Diels-Alder erhaltene 4-M-Δ4-THPSA besaß einen Schmelzpunkt von 68°C. Es enthielt 0,3 Gew.% organische Säuren und 60 mg/1 Wasser.

1) 4-M-Δ4-THPSA wurde 1 Stunde mit 7 Gew.% Montmorillonit-katalysator K 10 der Firma Südchemie, München, auf 150°C unter trockenem Stickstoff erhitzt. Nach Abfiltrieren des Katalysators und Abkühlung in trockener Atmosphäre besaß das lagerstabile, schwach gelbe Öl einen Erstarrungspunkt von - 5°C und enthielt 1,2 Gew.% organische Säuren, sowie 29 mg/1 Wasser.

0106968

- 8 -     O.Z. 3847-H

Die gaschromatografische Analyse ergab folgende identifizierbare Verbindungen:

| | |
|---|---|
| 4-M-$\Delta$4-THPSA | 26,5 Gew.% |
| Isomeres (Peak A) | 57,2 " " |
| Isomeres (Peak B) | 8,8 " " |
| Isomeres (Peak C) | 0,1 " " |
| 4-Methylphthalsäureanhydrid | - " " |

2) Der Versuch A.1) wurde mit 10 Gew.% KP-10-Montmorillonitkatalysator der Südchemie wiederholt, der zuvor bis zur Gewichtskonstanz bei 195$^{\circ}$C getrocknet worden war.

Das isolierte lagerstabile gelbe Öl hatte einen Schmelzpunkt von -8$^{\circ}$C. Es enthielt 0,2 Gew.% organische Säure und 55 mg/l Wasser. Die gaschromatografische Analyse zeigte folgende Zusammensetzung:

| | |
|---|---|
| 4-M-$\Delta$4-THPSA | 24,3 Gew.% |
| Isomeres (Peak A) | 61,1 " " |
| Isomeres (Peak B) | 8,1 " " |
| Isomeres (Peak C) | 0,1 " " |
| 4-Methylphthalsäureanhydrid | - " " |

3) Vergleichsversuch nach DE-PS 1 595 458.

4-M-$\Delta$4-THPSA wurde durch 7-stündiges Erhitzen auf 200$^{\circ}$C mit 0,2 Gew.% Palladium-Mohr isomerisiert. Nach Abtrennen des Katalysators besaß das erhaltene orangegelbe Öl einen Erstarrungspunkt von -4$^{\circ}$C. Nach zwei Wochen Lagerzeit hatten sich Kristalle aus 4-Methylphthalsäureanhydrid abgeschieden.

Die Gaschromatographie zeigte folgende Zusammensetzung:

0106968

| | |
|---|---|
| 4-M-Δ4-THPSA | 8,2 Gew.% |
| Isomeres (Peak A) | 32,2 " " |
| Isomeres (Peak B) | 3,9 " " |
| Isomeres (Peak C) | 32,1 " " |
| 4-Methylphthalsäureanhydrid | 7,2 " " |
| 4-Methylhexahydrophthalsäureanhydrid | 14,1 " " |

4) 2 kg 4-M-Δ4-THPSA (1,2 Gew.% organische Säure, 80 mg/l Wasser) wurde in 8 kg Hexahydrophthalsäureanhydrid (0,3 Gew.% organische Säure, 60 mg/l Wasser) gelöst. 1 kg dieser Lösung legte man in den 2,5 l fassenden Kolben einer Destillationsapparatur vor und versetzte sie mit 100 g Montmorillonitkatalysator KP 10. Die Destillationsapparatur war mit einer Kolonne mit 5 praktischen Böden und einem auf 90°C geheizten Dephlegmator bestückt. Nach Anlegen eines Vakuums von 56 mbar wurde die Mischung eine halbe Stunde am Rückfluß gekocht und das Destillat am Fuß der Kolonne abgenommen, während das Wasser über den Dephlegmator abgetrennt wurde. Nun gab man eine dem abdestillierten Produkt entsprechende Menge Lösung laufend hinzu und destillierte mit einer Geschwindigkeit von 1/2 kg/h Erzeugnis. Dabei stellte sich eine Sumpftemperatur von etwa 185°C ein. Das Destillat war ein wasserklares, farbloses Öl mit einem Erstarrungspunkt von - 10°C. Es enthielt weniger als 0,05 Gew.% organische Säuren und eine Wassermenge von 22 mg/l. Die gaschromatographische Analyse zeigte folgende Zusammensetzung:

| | |
|---|---|
| Hexahydrophthalsäureanhydrid | 79,8 Gew.% |
| 4-M-Δ4-THPSA | 5,8 " " |
| Isomeres (Peak A) | 12,8 " " |
| Isomeres (Peak B) | 0,9 " " |
| Isomeres (Peak C) | 0,1 " " |
| Phthalsäureanhydrid | - " " |
| 4-Methylphthalsäureanhydrid | - " " |
| Methylhexahydrophthalsäureanhydrid | - " " |

B) Isomerisierung von $\Delta$4-Tetrahydrophthalsäureanhydrid $\Delta$4-THPSA)

Durch Addition von Butadien an Maleinsäureanhydrid nach Diels-Alder wurde $\Delta$4-Tetrahydrophthalsäureanhydrid erhalten, das einen Schmelzpunkt von 103°C aufwies und 0,58 Gew.% organische Säuren, sowie 40 mg/l Wasser enthielt.

1) $\Delta$4-THPSA und 10 Gew.% Montmorillonit-Katalysator KP 10 der Firma Süd-Chemie, München, wurden unter Rühren und Stickstoffabdeckung auf 210°C erhitzt.

Nach Beendigung der Reaktion wurde der Katalysator abfiltriert. Das gewonnene, schwach gelbe Oel besaß einen Erstarrungspunkt von 0°C und enthielt 2,3 Gew.% organische Säuren, sowie 180 mg/l Wasser.
Das Gaschromatogramm zeigt das folgende Substanzspektrum.

| | |
|---|---|
| $\Delta$4-THPSA | 15,7 Gew.% |
| $\Delta$3-THPSA | 71,3  "   " |
| $\Delta$2-THPSA | 12,0  "   " |
| $\Delta$1-THPSA | -    "   " |
| Phthalsäureanhydrid | -    "   " |

2) Vergleichsversuch nach US-PS 2 764 597
Durch 4-stündiges Erhitzen auf 210°C mit 0,6 Gew.% Palladiumkohle (10% Pd) wurde $\Delta$4-THPSA isomerisiert. Nach Abtrennen des Katalysators wurde ein bei Zimmertemperatur festes Substanzgemisch gefunden. Die gaschromatografische Analyse hatte folgende Zusammensetzung:

| | |
|---|---|
| $\Delta$4-THPSA | 69,7 Gew.% |
| $\Delta$3-THPSA | 14,1 " " |
| $\Delta$2-THPSA | 10,2 " " |
| $\Delta$1-THPSA | 0,1 " " |
| Phthalsäureanhydrid | 1,0 " " |
| Hexahydrophthalsäureanhydrid | 2,1 " " |

C) Isomerisierung von 4-Methyl-$\Delta$4-tetrahydrophthal-säuredimethylester (4-M-$\Delta$4-THPE)

4-M-$\Delta$4-THPE wurde durch 3-stündiges Erhitzen auf 170°C unter Zusatz von 10 Gew.% Katalysator KP 10 der Firma Südchemie isomerisiert. Das KP 10 war zuvor bei 150°C bis zur Gewichtskonstanz getrocknet worden.

Das gewonnene, schwach gelbe Oel wurde durch Gaschromatographie untersucht.

| | |
|---|---|
| 4-M-$\Delta$4-THPE | 53,2 Gew.% |
| Isomeres (Peak I) | 34,5 " " |
| Isomeres (Peak II) | 7,1 " " |
| Isomeres (Peak III) | 1,0 " " |

D) Isomerisierung von $\Delta$4-Tetrahydrophthalsäuredimethyl-ester ($\Delta$4-THPE)

Mit $\Delta$4-THPE wurde sinngemäß, wie unter C) beschrieben, verfahren. Das Gaschromatogramm des gelben Oels zeigte anschließend folgende Werte:

| | |
|---|---|
| $\Delta$4-THPE | 45,5 Gew.% |
| $\Delta$3-THPE | 44,8 " " |
| $\Delta$2-THPE | 3,5 " " |

0106968

O.Z. 3847-H

Patentanspruch:

Verfahren zur Herstellung von Isomeren der 4-Methyl-tetra-hydrophthalsäurederivate und/oder Tetrahydrophthalsäurede-rivate, insbesondere deren Anhydride, gegebenenfalls im Gemisch mit Methyl-hexahydrophthalsäure-Verbindungen und/oder Hexahydrophthalsäure-Verbindungen, dadurch gekennzeichnet, daß man die 4-Methyl-Δ4-tetrahydrophthalsäure-derivate und/oder 4-Tetrahydrophthalsäurederivate bei Temperaturen von 130°C - 250°C, insbesondere bei 150°C - 220°C, mit aktivierten Bleicherden behandelt.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| X | DE-A-1 518 509  (AIR PRODUCTS) * Ansprüche 1-5; Seite 7, Absatz 2 * | 1 | C 07 C  61/24 C 07 C  51/567 C 07 C  51/353 C 07 C  69/75 C 07 C  67/333 |
| | --- | | |
| X | US-A-3 470 214  (YOUNG) * Ansprüche 1,5,7,9-13 * | 1 | |
| | ----- | | |

| | |
|---|---|
| | RECHERCHIERTE SACHGEBIETE (Int. Cl. ³) |
| | C 07 C  61/00 C 07 C  51/00 |

Der vorliegende Recherchenbericht wurde für alle Patentansprüche erstellt.

| Recherchenort | Abschlußdatum der Recherche | Prüfer |
|---|---|---|
| DEN HAAG | 13-01-1984 | KLAG M.J. |